Europäisches Patentamt

European Patent Office

Office européen des brevets

(19)

(11) Numéro de publication : **0 362 764 B1**

# (12) FASCICULE DE BREVET EUROPEEN

(45) Date de publication du fascicule du brevet :
**19.02.92 Bulletin 92/08**

(51) Int. Cl.$^5$ : **A61F 9/00**

(21) Numéro de dépôt : **89118230.5**

(22) Date de dépôt : **02.10.89**

(54) **Dispositif générateur d'enveloppe d'un faisceau de puissance.**

(30) Priorité : **06.10.88 CH 3746/88**
**10.10.88 FR 8813398**

(43) Date de publication de la demande :
**11.04.90 Bulletin 90/15**

(45) Mention de la délivrance du brevet :
**19.02.92 Bulletin 92/08**

(84) Etats contractants désignés :
**AT BE DE GB IT LU NL SE**

(56) Documents cités :
**EP-A- 0 030 210**
**GB-A- 2 150 315**
**US-A- 3 710 798**

(73) Titulaire : **LASAG AG**
**Mittlere Strasse 52**
**CH-3600 Thun (CH)**

(72) Inventeur : **Taoufik, Nouri**
**Oberstadelstrasse 14**
**CH-3653 Oberhofen (CH)**

(74) Mandataire : **de Raemy, Jacques et al**
**ICB Ingénieurs Conseils en Brevets SA**
**Passage Max. Meuron 6**
**CH-2001 Neuchâtel (CH)**

**Description**

La présente invention est relative à un dispositif de traitement chirurgical, notamment en ophtalmologie, comprenant :

– au moins une première source de lumière émettant un faisceau de puissance selon un premier axe optique et selon une section déterminée,

– une seconde source de lumière émettant un faisceau de lumière visible selon un deuxième axe optique,

– un équipage mobile en rotation autour dudit deuxième axe optique, ledit équipage comportant des moyens pour transformer le faisceau issu de ladite seconde source en au moins un faisceau élémentaire tournant autour dudit deuxième axe optique et arrangé pour former un faisceau lumineux tubulaire destiné à envelopper ledit faisceau de puissance,

– des moyens pour combiner ledit faisceau de puissance et ledit faisceau lumineux tubulaire selon un troisième axe optique commun, et

– une lentille disposée sur le trajet dudit troisième axe optique pour concentrer ledit faisceau de puissance et ledit faisceau lumineux tubulaire qui l'entoure sur le point de focalisation de la lentille, selon un cône d'angle au sommet déterminé.

La présente invention est également relative à un dispositif comme défini ci-dessus mais dans lequel la première source de lumière émet un faisceau de puissance selon une section qui peut être variée.

De tels dispositif sont décrits dans le brevet EP-B-0 030 210. Ces dispositifs, incorporés à un appareil pour l'observation en vue du traitement d'un oeil par chirurgie laser, permettent, non seulement de visualiser le faisceau de puissance invisible en lui-même, mais aussi de vérifier qu'aucun obstacle (tel que l'iris par exemple) ne se trouve sur le trajet dudit faisceau.

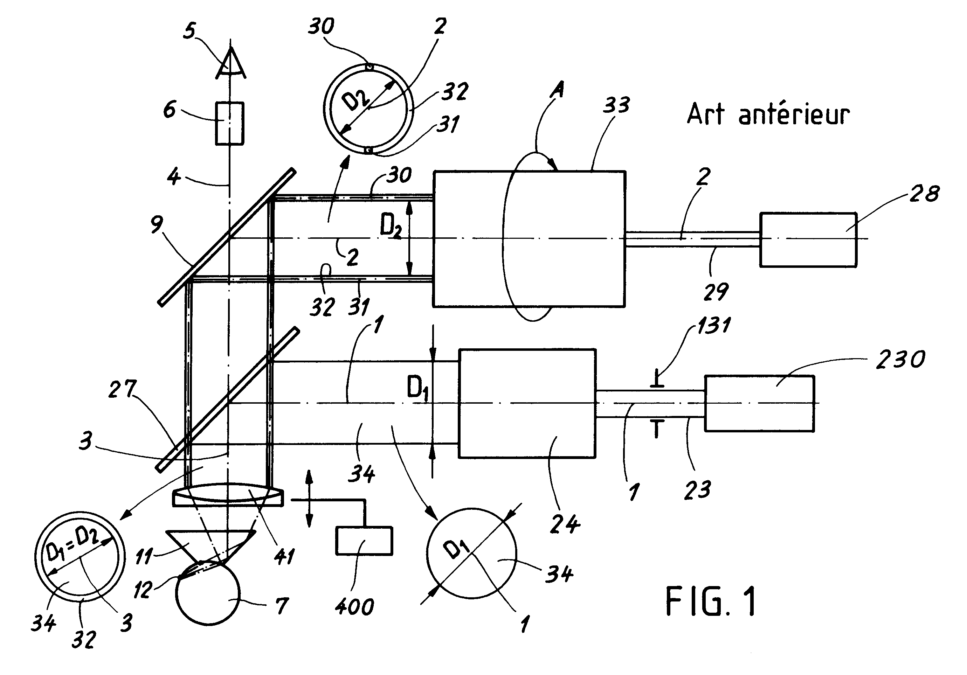

Cet art antérieur sera brièvement rappelé maintenant. On consultera pour cela la figure 1 de la présente description.

L'appareil comporte une première source de lumière 230 qui émet un faisceau de puissance 23 destiné au traitement chirurgical. Ce faisceau est émis selon un premier axe optique 1 à partir, par exemple, d'une source laser à corps solide fonctionnant par impulsions (par exemple en mode Q switch). La section du faisceau peut être modifiée soit au moyen d'un diaphragme à iris 131, soit au moyen d'une optique d'adaptation symbolisée ici par le bloc 24. A la sortie de ces éléments, le faisceau se propageant selon l'axe 1 présente une section déterminée. Cette section est ici cylindrique de diamètre $D_1$. Mais, de façon connue, elle pourrait être par exemple carrée ou rectangulaire.

L'appareil comporte encore une seconde source de lumière 28 qui émet un faisceau de lumière visible 29 de faible puissance. Ce faisceau est émis selon un deuxième axe optique à partir, par exemple, d'un laser He-Ne. Le faisceau 29 est reçu par un dispositif comportant un équipage mobile 33 tournant, par exemple dans le sens de la flèche A, autour du deuxième axe optique 2. Cet équipage comporte, dans le document cité, un ensemble de prismes et de miroirs transformant le faisceau 29 en deux faisceaux élémentaires parallèles 30 et 31. Quand l'équipage 33 tourne, les faisceaux élémentaires engendrent un faisceau lumineux tubulaire 32 de diamètre $D_2$ et coaxial au deuxième axe optique 2. L'équipage mobile est arrangé de telle façon que le diamètre $D_2$ du faisceau tubulaire 32 soit égal au diamètre $D_1$ du faisceau de puissance 34. Cette adaptation est réalisée dans le document cité par un levier à glissière commandant deux prismes. De chacun de ces prismes est émis un faisceau élémentaire, lesdits faisceaux étant susceptibles de s'écarter ou de s'éloigner quand on manoeuvre le levier.

La figure 1 montre encore que l'appareil est muni de deux miroirs semi-réfléchissants 9 et 27 qui ont pour but de combiner le faisceau de puissance 34 et le faisceau lumineux tubulaire 32 selon un troisième axe optique 3 de telle manière que le faisceau 32 entoure le faisceau 34.

Sur le trajet du troisième axe optique 3 on dispose une lentille 41 qui peut être déplacée axialement au moyen d'un mécanisme 400 qui sert à ajuster le point focal de la lentille sur l'endroit à traiter, respectivement à observer.

Ainsi, à la sortie de la lentille 41, on trouve un cône lumineux dont l'angle au sommet est déterminé, d'une part, par la distance focale de la lentille et, d'autre part, par les diamètres $D_1$ et $D_2$ du faisceau 34 et du faisceau 32 respectivement. Le cône du faisceau 34 est entouré alors par un cône enveloppe issu du faisceau 32.

Le point de focalisation de la lentille 41 peut être observé par un praticien 5. L'oeil du praticien s'appuie sur un complexe optique 6 qui définit un faisceau d'observation 4 se superposant en fin de compte à l'axe optique 3. Ce complexe peut aussi être un système binoculaire.

Le cône lumineux de sortie de la lentille 41 est dévié par une lentille de contact 11 que le praticien appuie sur l'oeil 7 du patient de telle façon que le point focal 12 de ladite lentille tombe à l'endroit désiré.

Le lecteur se référera au document cité pour obtenir encore plus de détails sur le fonctionnement de tout le système. Que l'on rappelle simplement ici que le faisceau d'observation est pourvu d'une grande profondeur de champ et que, par voie de conséquence l'observation visuelle est impropre à situer avec précision la position du foyer de la lentille 41, ce qui explique la présence de la seconde source de lumière 28 qui permet une mise au point extrêmement sensible du foyer du faisceau de puissance 34. On notera en passant que cette seconde source peut être cohérente ou non cohérente.

Comme on l'a dit ci-dessus l'équipage mobile 33 comporte un ensemble de prismes et de miroirs. Or, la fabrication, le montage et le réglage de tels éléments sont extrêmement coûteux. De plus, un tel équipement est lourd et volumineux, ce qui accroît le poids et l'encombrement du dispositif.

Pour remédier à ces inconvénients, la présente invention propose de supprimer l'ensemble de prismes et de miroirs faisant partie de l'équipage mobile de telle sorte qu'elle est caractérisée, selon un premier mode d'exécution, par le fait que les moyens pour transformer le faisceau issu de la seconde source lumineuse en au moins un faisceau élémentaire tournant autour du deuxième axe optique comportent au moins un guide homogène de lumière.

Comme on l'a également mentionné ci-dessus, le dispositif selon l'art antérieur comporte une adaptation possible du diamètre du faisceau lumineux tubulaire au diamètre du faisceau de puissance. Cette adaptation est faite hors de l'équipage en rotation au moyen de prismes et d'un levier commandant ces prismes. Ce système présente aussi l'inconvénient d'être difficile à fabriquer, à monter et à régler.

Aussi pour remédier à ces inconvénients, la présente invention propose d'inclure à l'équipage mobile le dispositif permettant l'adaptation des diamètres. Dans ce but, elle comporte, en plus des caractéristiques propres au premier mode d'exécution, des seconds moyens faisant partie de l'équipage mobile adaptés pour faire varier le diamètre que présente le faisceau lumineux tubulaire de manière à ce qu'il enveloppe le faisceau de puissance en correspondance avec la section choisie pour ledit faisceau de puissance.

En résumé et grâce aux caractéristiques données ci-dessus, le dispositif selon l'invention permet d'employer des moyens plus légers, plus souples, moins fragiles et moins chers que ceux utilisés dans la technique antérieure sans perdre pour autant, ni en précision, ni en fiabilité.

L'invention sera comprise maintenant à l'aide de la description qui va suivre, donnée uniquement à titre d'exemple, et en se référant au dessin dans lequel :

La figure 1 est un croquis schématique illustrant l'art antérieur expliqué ci-dessus.

La figure 2 est une coupe à travers l'équipage mobile référencé 33 en figure 1 et réalisé selon un premier mode d'exécution de l'invention.

La figure 3 est une coupe à travers l'équipage mobile référencé 33 en figure 1 et réalisé selon une variante du premier mode d'exécution de l'invention.

La figure 4 est une coupe à travers l'équipage mobile référencé 33 en figure 1 et réalisé selon un second mode d'exécution de l'invention.

La figure 5 est une vue selon la flèche V de la figure 4.

La figure 6 est une coupe à travers l'équipage mobile référencé 33 en figure 1 et réalisé selon une première variante du second mode d'exécution de l'invention.

La figure 7 est une vue selon la flèche VII de la figure 6.

La figure 8 est une coupe à travers l'équipage mobile référencé 33 en figure 1 et réalisé selon une seconde variante du second mode d'exécution de l'invention.

La figure 9 est une vue selon la flèche IX de la figure 8.

La figure 10 est une coupe partielle à travers l'équipage mobile référencé 33 en figure 1 et réalisé selon une troisième variante du second mode d'exécution de l'invention.

Les figures 11 et 12 sont des coupes partielles illustrant un atténuateur à lentille placé à l'entrée de l'équipage mobile référencé 33 en figure 1, pour deux positions relatives de cet atténuateur.

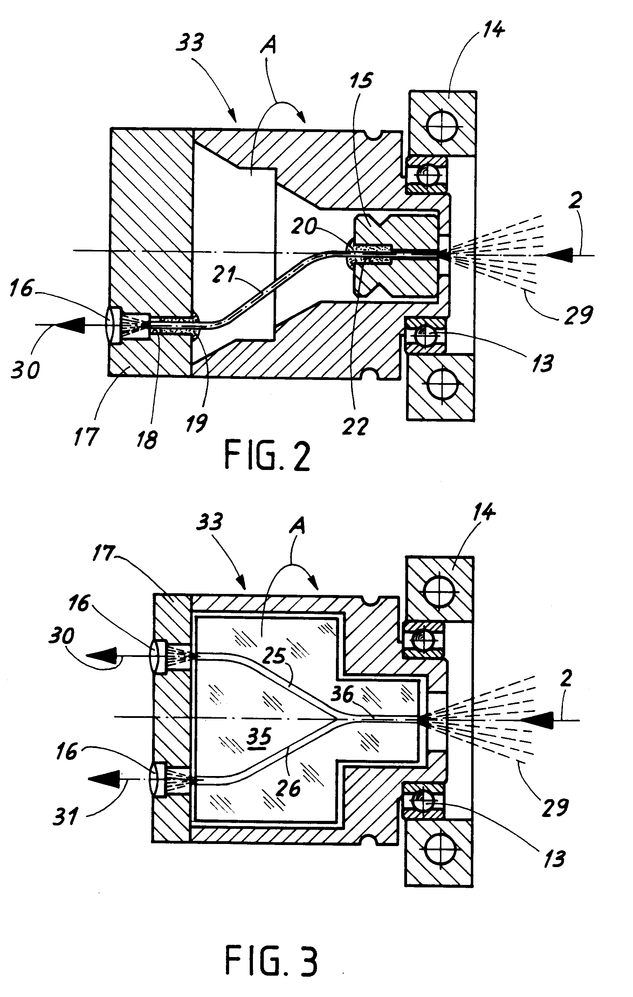

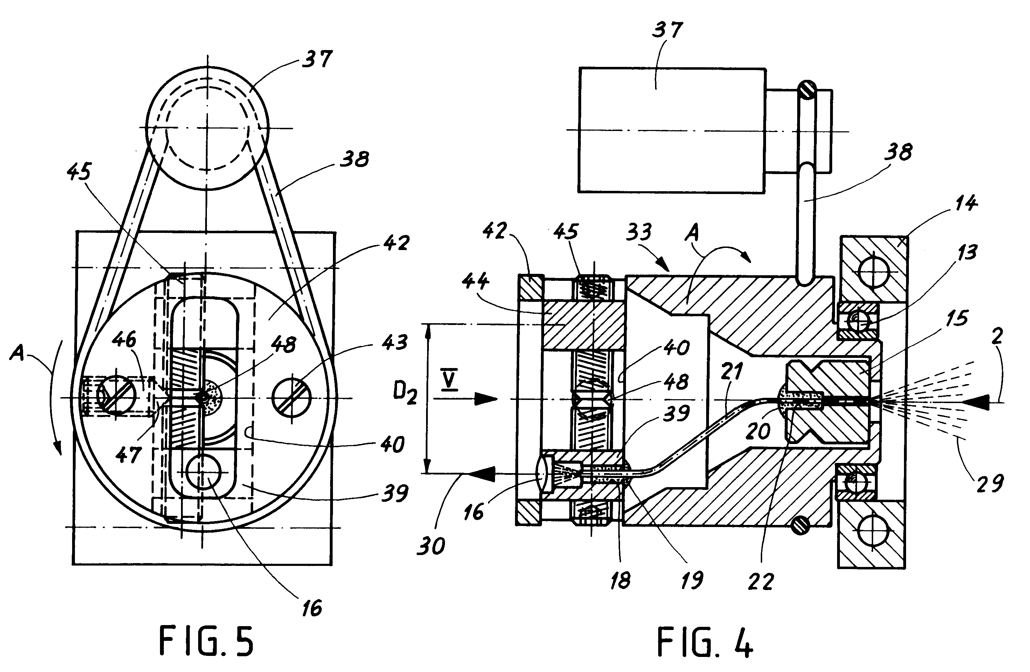

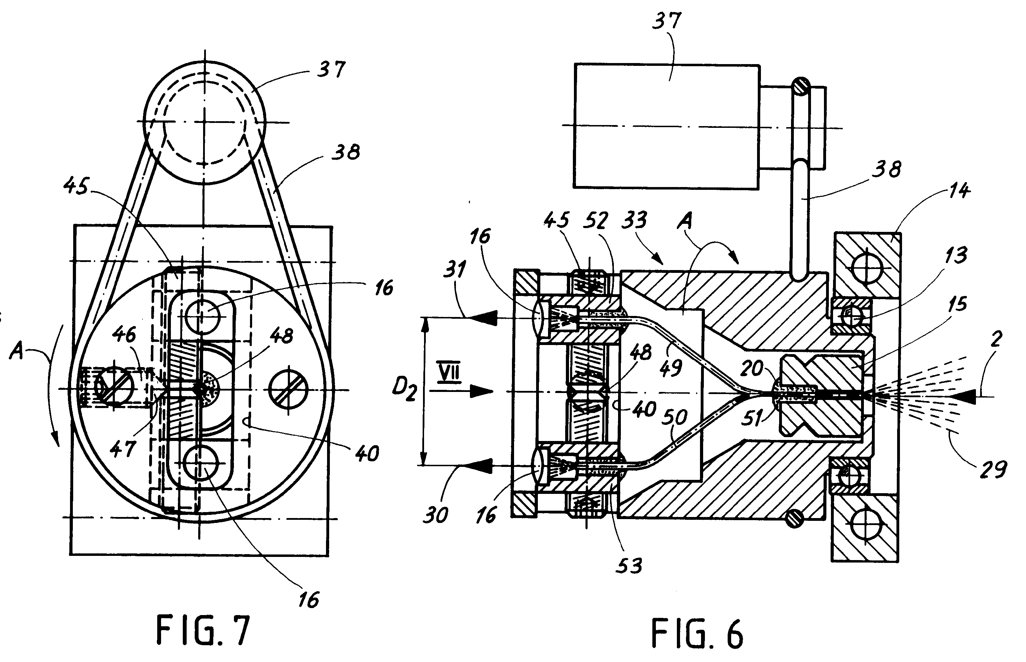

L'équipage mobile 33 montré en figure 2 est un tambour tournant dans le sens de la flèche A. Cet équipage 33 s'inserre dans le schéma de la figure 1 à la place du bloc portant la même référence. Des moyens sont mis en oeuvre pour entraîner le tambour en rotation, moyens qui seront expliqués plus loin, par exemple à l'aide des figures 4 et 5. Le tambour tourne sur un bâti fixe 14 dont il est solidaire, par l'intermédiaire d'un roulement à billes 13. L'axe de rotation du tambour est confondu avec le deuxième axe optique 2, ce deuxième axe étant celui du faisceau de lumière 29 émis par la seconde source de lumière 28, comme cela a été expliqué à propos de la figure 1. Selon l'invention, le faisceau 29 pénètre dans un guide homogène de lumière 21. Comme le montre la figure 2, le faisceau 29 est transformé en un faisceau élémentaire 30 décalé par rapport au deuxième axe 2 et parallèle à cet axe. Quand le tambour tourne, le faisceau élémentaire 30 engendre un faisceau lumineux tubulaire 32 (voir figure 1). Une extrémité du guide 21 pénètre dans un orifice 22 percé dans un élément cylindrique 15 solidaire du tambour, extrémité qui reçoit le faisceau 29. L'autre extrémité du guide 21 pénètre dans un autre orifice 18 percé dans la paroi frontale 17 du tambour. Dans l'orifice 18 est placée une lentille 16 destinée à rendre parallèle la lumière émergeant du guide 21 et formant le faisceau élémentaire 30. Le guide 21 est maintenu dans les orifices 18 et 22 au moyen de points de colle référencés respectivement 19 et 20. Dans le cas particulier montré en figure 2, le guide homogène de lumière 21 n'est pas autre chose qu'une simple fibre optique, bien connue pour elle-même, par exemple dans le domaine des télécommunications. Dans ce cas précis, on voit que le faisceau lumineux tubulaire 32 (figure 1) est engendré par un seul faisceau élémentaire 30 produit par un unique guide 21, ledit faisceau élémentaire tournant autour de l'axe 2.

La figure 3 est une coupe à travers l'équipage mobile 33 réalisé selon une variante du premier mode d'exécution de l'invention décrite ci-dessus. Dans ce cas, le tambour comporte des moyens pour transformer le faisceau 29 en deux faisceaux élémentaires 30 et 31, ces moyens consistant à utiliser deux guides homogènes de lumière 25 et 26. Dans cette exécution particulière, on dispose à l'intérieur du tambour une plaque de verre 35 sur laquelle on forme des canaux 25, 26 et 36 par dopage de ladite plaque, ce dopage pouvant être réalisé au moyen de germanium ou de sel de potassium par exemple. Le canal 36 se divise ici en deux branches 25 et 26. L'entrée du canal 36 reçoit le faisceau 29 et, à chacune des sorties des canaux 25 et 26, on dispose une lentille 16 permettant, comme cela a déjà été dit, de rendre parallèle la lumière émergeant de ces canaux. En tournant le tambour engendre alors un faisceau lumineux tubulaire 32 (figure 1) issu cette fois-ci de deux faisceaux élémentaires 30 et 31 diamétralement opposés et tournant à égale distance du deuxième axe optique 2. Ce mode de faire est généralement préféré au mode qui n'utilise qu'un faisceau élémentaire, pour la plus grande précision qu'il apporte.

En variante à l'exécution présentée en figure 3, on pourrait avoir, au lieu des canaux 25, 26 et 36 dopés dans la plaque de verre 35, des fibres optiques présentant la même configuration que lesdits canaux et disposées dans le tambour de façon similaire à ce qui a été montré en figure 2. De telles fibres optiques sont connues sous le nom de coupleur en télécommunication.

Le premier mode d'exécution de l'invention décrit à l'aide des figures 2 et 3 ne permet d'engendrer qu'un seul faisceau lumineux tubulaire. Ce mode ne s'applique donc que dans le cas où l'on n'envisage qu'un seul faisceau de puissance dont la section est fixe et bien déterminée. Dans de très nombreux cas cependant, le chirurgien doit pouvoir choisir entre l'utilisation de faisceaux de puissance présentant au moins deux sections différentes, la dimension du faisceau définissant un cône d'angle au sommet déterminé, après que ledit faisceau ait traversé la lentille convergente 41 (figure 1). Un cône d'angle au sommet relativement grand, par exemple 16°, est préféré pour la profondeur de champ réduite présentée par sa distance focale et donc la précision d'emplacement du point où doit s'opérer le traitement chirurgical. Un tel angle est également préféré dans le cas de l'utilisation d'un laser à impulsions développant une grande puissance. Quelques fois par contre, un tel angle d'ouverture empêche d'atteindre certains endroits de l'oeil, comme par exemple celui représenté en figure 1 qui est proche de l'angle irido-cornéen. Dans ce cas, il est nécessaire de réduire l'angle d'ouverture du cône de traitement de moitié, soit 8° par exemple.

La modification de l'angle d'ouverture du cône de traitement est réalisé, comme on l'a dit à propos de la figure 1, par un diaphragme 131 ou une optique 24 qui modifie la section du faisceau de puissance 34 qui se présente ici sous la forme d'un cylindre de diamètre $D_1$ avant son passage dans la lentille convergente 41. Corrélativement à cela il est donc nécessaire de modifier le diamètre $D_2$ du faisceau lumineux tubulaire qui doit rester l'enveloppe du faisceau de puissance. On en arrive alors au second mode d'exécution de l'invention qui permet cette modification, tout en maintenant l'utilisation de guides homogènes de lumière, objet du premier mode d'exécution de l'invention. Deux cas peuvent se présenter, selon que cette modification du diamètre $D_2$ est réalisée de manière continue ou par paliers.

Les figures 4 et 5 montrent un équipage mobile 33 arrangé pour faire varier de manière continue le diamètre $D_2$ du faisceau lumineux tubulaire en correspondance avec le diamètre $D_1$ choisi pour le cylindre du faisceau de puissance. Ce deuxième mode d'exécution de l'invention comporte à nouveau un tambour tournant dans le sens de la flèche A et s'insérant dans le schéma de la figure 1 à la place du bloc portant la référence 33. La rotation du tambour est assurée par un moteur 37 couplé au tambour au moyen d'une courroie 38. Le tambour tourne sur un bâti fixe 14 dont il est solidaire, par l'intermédiaire d'un roulement à billes 13. Le faisceau de lumière 29 émis par la seconde source de lumière 28 (figure 1) pénètre dans un guide homogène de lumière 21. De cette façon, le faisceau 29 est transformé en un faisceau élémentaire 30 décalé par rapport à l'axe de rotation 2 du tambour. Quand le tambour tourne, le faisceau élémentaire 30 engendre un faisceau lumineux tubulaire 32 (figure 1).

Une extrémité du guide 21 pénètre dans un orifice 22 percé dans un élément cylindrique 15 solidaire du tambour. L'autre extrémité du guide 21 pénètre dans un autre orifice 18 percé dans un véhicule 39 susceptible de coulisser dans une glissière 40. La glissière 40 est formée, d'une part, par une rainure pratiquée dans le tambour et, d'autre part, par un couvercle 42 qui recouvre le tambour et qui y est fixé au moyen de vis 43. La glissière 40 comporte un autre véhicule 44 qui est la réplique du premier véhicule mais qui n'est pas pourvu d'orifice. On fait coulisser les véhicules dans la glissière en actionnant une vis sans fin 45 reçue dans des taraudages correspondants pratiqués dans les véhicules. Comme le montrent les figures 4 et 5, la vis sans fin comporte un pas qui s'inverse au milieu de ladite vis. De cette façon, on comprend que lorsqu'on tourne la vis 45 dans un sens les véhicules s'écartent et lorsqu'on tourne cette vis dans l'autre sens les véhicules se rapprochent l'un de l'autre. Une vis de blocage 46 permet de fixer la position des véhicules quand on a choisi le diamètre de travail du faisceau lumineux tubulaire. A cet effet, la vis 46 se termine par une pointe 47 susceptible de pénétrer dans une gorge 48 pratiquée dans la vis sans fin.

Comme cela était déjà le cas pour le véhicule de la figure 2, le guide 21 est ici une fibre optique assujettie à l'élément 15 et au véhicule 39 au moyen de points de colle référencés respectivement en 20 et 19. De la même façon, le véhicule 39 comporte une lentille 16 destinée à rendre parallèle la lumière émise par le guide 21 et formant le faisceau élémentaire 30.

Les figures 6 et 7 montrent une première variante du second mode d'exécution de l'invention. Ici, l'équipage mobile 33 comporte deux fibres optiques couplées présentant la forme d'un Y. La branche inférieure 51 de l'Y, assujettie à l'élément 15 par le point de colle 20, reçoit le faisceau de lumière 29, alors que chacune des branches supérieures 49 et 50 de l'Y est reçue par un véhicule 52, 53 monté dans l'équipage mobile 33 de la même façon que cela a été décrit à propos des figures 4 et 5. On produit ainsi deux faisceaux élémentaires 30 et 31 qui engendrent, quand le tambour tourne, un faisceau lumineux tubulaire 32 (figure 1), le diamètre $D_2$ de ce faisceau pouvant être varié en actionnant la vis sans fin 45.

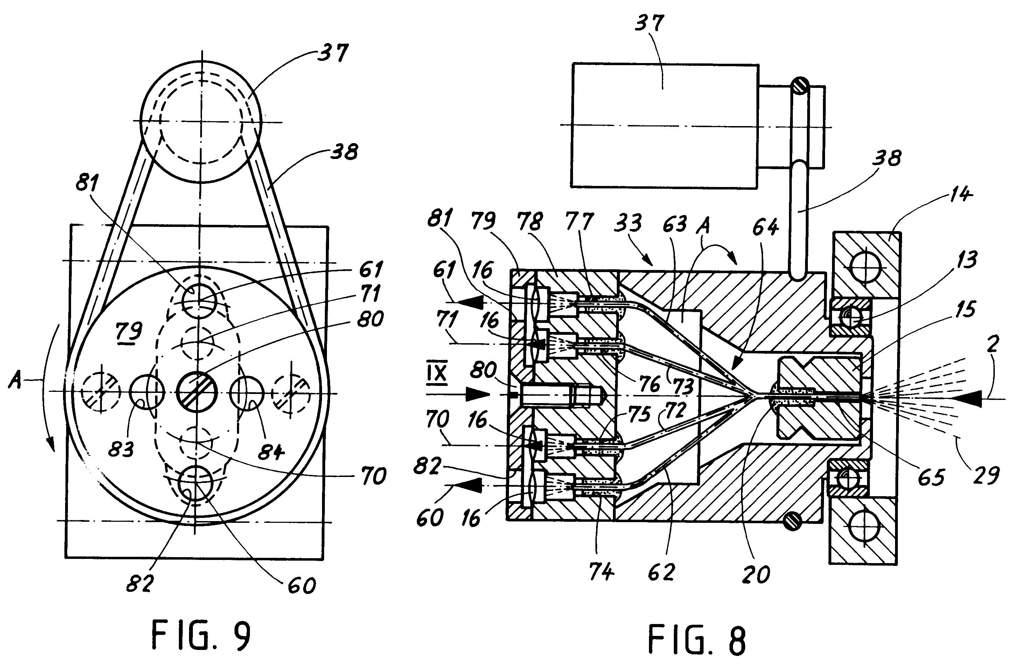

Les figures 8 et 9 montrent une deuxième variante du second mode d'exécution de l'invention. Cette variante permet de choisir deux diamètres $D_2$ différents pour le faisceau lumineux tubulaire, par exemple un petit diamètre pour produire un cône d'angle au sommet de 8° et un grand diamètre pour produire un cône d'angle au sommet de 16° (valeurs évoquées plus haut). A cet effet, l'équipage mobile 33 comporte des premiers moyens pour transformer le faisceau 29 issu de la seconde source de lumière en première 60, 61 et en seconde 70, 71 paires de faisceaux élémentaires. Ces moyens comportent un guide homogène de lumière 64 se développant de manière arborescente comme on le voit bien en figure 8. Les faisceaux élémentaires 60, 61, engendrant un faisceau lumineux tubulaire de grand diamètre, sont produits par les guides homogènes 62, 63 et les faisceaux élémentaires 70, 71, engendrant un faisceau lumineux tubulaire de petit diamètre, sont produits par les guides homogènes 72, 73. Le guide 64 est ici une fibre optique du genre de celle utilisée par exemple en télécommunication et connue sous le nom de coupleur. L'extrémité 65 du guide 64 est fixée de la manière déjà décrite à un élément cylindrique 15 fixé à l'équipage 33. Cette extrémité 65 reçoit le faisceau 29. Les autres extrémités des guides 62, 63, 72 et 73 sont assujetties dans des orifices référencés 74 à 77 et percés dans une plaque de fermeture 78. Dans l'alignement de ces orifices on trouve des lentilles 16 dont la fonction a été expliquée ci-dessus.

Les moyens de sélection entre le faisceau tubulaire engendré par les faisceaux élémentaires 60 et 61 et le faisceau tubulaire engendré par les faisceaux élémentaires 70 et 71, consistent à disposer devant la plaque 78 un disque 79 percé de quatre trous, le disque étant maintenu sur la plaque au moyen d'une vis 80. Si l'on choisit les faisceaux 60 et 61, on oriente le disque 79 de manière à ce que les trous 81 et 82 laissent passer ces faisceaux comme cela est représenté en figures 8 et 9. Si l'on choisit les faisceaux 70 et 71, on fera tourner le disque 78 de 90°, auquel cas les faisceaux 70 et 71 pourront passer par les trous 83 et 84, les faisceaux 60 et 61 étant alors obturés.

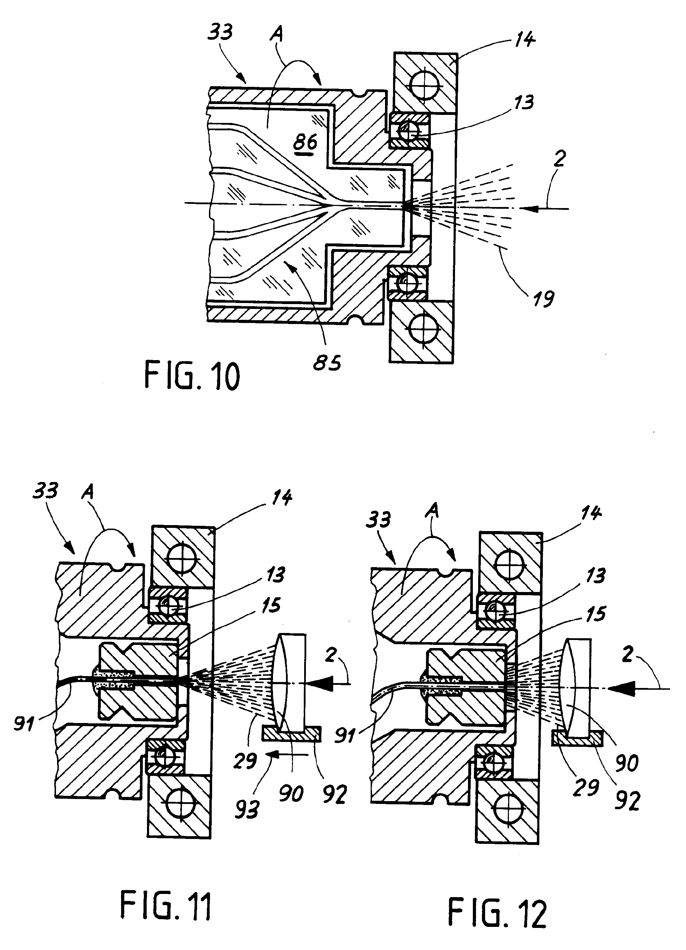

Une troisième variante du second mode d'exécution de l'invention est montré dans la coupe partielle de la figure 10. Ici la fibre optique 64 de la figure 8 est remplacée par des canaux 85 obtenus par dopage d'une plaque de verre 86. Cette construction est fortement apparentée à celle discutée à propos de la figure 3. Le résultat obtenu est cependant le même que celui exposé à propos des figures 8 et 9. On retrouvera ici les mêmes moyens de sélection, c'està-dire la mise en oeuvre d'une plaque percée de quatre trous.

Dans certains cas d'utilisation de l'appareil décrit ici, on souhaite pouvoir varier l'intensité lumineuse émise par les faisceaux élémentaires sortant de l'équipage mobile. Cela peut être réalisé au moyen d'un atténuateur situé entre la seconde source de lumière 28 et l'équipage mobile 33 (figure 1).

Avantageusement cet atténuateur est un véhicule 92 portant une lentille 90 dont les rayons de sortie convergent vers l'équipage mobile, comme cela est représenté en figures 11 et 12. Le faisceau 29 issu de la source 28 (figure 1) est rendu convergent par la lentille 90. Ce faisceau pénètre l'extrémité du guide de lumière 91 réalisé de l'une quelconque des façons décrites ci-dessus. La figures 11 montre que tout le faisceau 29 peut pénétrer dans le guide 91. On obtiendra à ce moment une intensité maximale de lumière. Si l'on défocalise la lentille 90 en entraînant le véhicule dans le sens de la flèche 93, une partie seulement de la lumière atteindra le guide 91, comme on le voit en figure 12. On a réalisé de cette manière un atténuateur de lumière très simple et efficace.

## Revendications

1. Dispositif de traitement chirurgical, notamment en ophtalmologie, comprenant :
   – au moins une première source de lumière (230) émettant un faisceau de puissance (23) selon un premier axe optique (1) et selon une section (34) déterminée,
   – une seconde source de lumière (28) émettant un faisceau de lumière visible (29) selon un deuxième axe optique (2),
   – un équipage mobile (33) en rotation autour dudit deuxième axe optique, ledit équipage comportant des moyens pour transformer le faisceau issu de ladite seconde source en au moins un faisceau élémentaire (30) tournant autour dudit deuxième axe optique et arrangé pour former un faisceau lumineux tubulaire (32) destiné à envelopper ledit faisceau de puissance,

– des moyens (9, 27) pour combiner ledit faisceau de puissance et ledit faisceau lumineux tubulaire selon un troisième axe optique commun (3), et

– une lentille (41) disposée sur le trajet dudit troisième axe optique pour concentrer ledit faisceau de puissance et ledit faisceau lumineux tubulaire qui l'entoure sur le point de focalisation (12) de la lentille, selon un cône d'angle au sommet déterminé,

caractérisé par le fait que lesdits moyens faisant partie dudit équipage mobile comportent au moins un guide homogène de lumière (21).

2. Dispositif selon la revendication 1, caractérisé par le fait que le guide homogène de lumière est une fibre optique.

3. Dispositif selon la revendication 1, caractérisé par le fait que ledit équipage mobile comporte des moyens pour transformer le faisceau issu de ladite seconde source en deux faisceaux élémentaires (30, 31) et que lesdits moyens comprennent deux guides homogènes de lumière.

4. Dispositif selon la revendication 3, caractérisé par le fait que les guides homogènes de lumière sont des fibres optiques couplées.

5. Dispositif selon la revendication 3, caractérisé par le fait que les guides homogènes de lumières sont des canaux (25, 26, 36) obtenus par dopage d'une plaque de verre (35).

6. Dispositif de traitement chirurgical, notamment en ophtalmologie, comprenant :

– au moins une première source de lumière (230) émettant un faisceau de puissance (23) selon un premier axe optique (1) et selon une section (34) qui peut être variée,

– une seconde source de lumière (28) émettant un faisceau de lumière visible (29) selon un deuxième axe optique (2),

– un équipage mobile (33) en rotation autour dudit deuxième axe optique, ledit équipage comportant des premiers moyens pour transformer le faisceau issu de ladite seconde source en au moins un faisceau élémentaire (30) tournant autour dudit deuxième axe optique et arrangé pour former un faisceau lumineux tubulaire (32) destiné à envelopper ledit faisceau de puissance,

– des moyens (9, 27) pour combiner ledit faisceau de puissance et ledit faisceau lumineux tubulaire selon un troisième axe optique commun (3), et

– une lentille (41) disposée sur le trajet dudit troisième axe optique pour concentrer ledit faisceau de puissance et ledit faisceau lumineux tubulaire qui l'entoure sur le point de focalisation (12) de la lentille, selon un cône d'angle au sommet déterminé,

caractérisé par le fait que les premiers moyens faisant partie de l'équipage mobile comportent au moins un guide homogène de lumière (21) et que ledit équipage mobile comporte en outre des seconds moyens adaptés pour faire varier le diamètre ($D_2$) que présente le faisceau lumineux tubulaire de manière à ce qu'il enveloppe ledit faisceau de puissance en correspondance avec la section ($D_1$) choisie pour ledit faisceau de puissance.

7. Dispositif selon la revendication 6, caractérisé par le fait que l'équipage mobile comporte des premiers moyens pour transformer le faisceau issu de ladite seconde source en deux faisceaux élémentaires (30, 31), lesdits premiers moyens comprenant deux fibres optiques couplées (49, 50, 51) présentant la forme d'un Y et que les seconds moyens comportent un arrangement permettant de rapprocher ou d'écarter les deux branches supérieures (49, 50) de l'Y l'une de l'autre de part et d'autre dudit deuxième axe optique.

8. Dispositif selon la revendication 7, caractérisé par le fait que ledit arrangement comporte deux véhicules (52, 53) portant chacun l'extrémité d'une fibre optique constituant une branche supérieure de l'Y, les véhicules étant susceptibles de coulisser dans une glissière (40) sous l'action d'une vis sans fin (45) à pas inversé au milieu de la longueur de ladite vis pour écarter ou pour rapprocher lesdits véhicules l'un de l'autre quand on tourne la vis sans fin respectivement dans un premier sens ou dans un second sens.

9. Dispositif selon la revendication 6, caractérisé par le fait que l'équipage mobile comporte des premiers moyens pour transformer le faisceau issu de ladite seconde source en première (60, 61) et seconde (70, 71) paires de faisceaux élémentaires, lesdits premiers moyens comprenant un guide homogène de lumière se développant de manière arborescente pour former, quand il est entraîné par l'équipage mobile en rotation des premier et second faisceaux lumineux tubulaires coaxiaux de diamètres différents et que lesdits seconds moyens comportent un élément permettant de sélectionner l'une ou l'autre desdites paires de faisceaux élémentaires.

10. Dispositif selon la revendication 9, caractérisé par le fait que le guide homogène de lumière se développant de manière arborescente est constitué par des fibres optiques (64).

11. Dispositif selon la revendication 9, caractérisé par le fait que le guide homogène de lumière se développant de manière arborescente est constitué par des canaux (85) obtenus par dopage d'une plaque de verre (86).

12. Dispositif selon la revendication 9, caractérisé par le fait que ledit élément de sélection comporte une plaque à trous (79) pouvant être disposée dans une première position pour laquelle elle laisse passer la première paire de faisceaux élémentaires ou dans une seconde position pour laquelle elle laisse passer la seconde paire de faisceaux élémentaires.

13. Dispositif selon la revendication 1 ou la revendication 6, caractérisé par le fait qu'entre la seconde source de lumière et l'équipage mobile est situé un atténuateur (90, 92) permettant de faire varier l'intensité de lumière émise par le ou les faisceaux élémentaires.

14. Dispositif selon la revendication 13, caractérisé par le fait que l'atténuateur est un véhicule (92) portant une lentille (90) dont les rayons de sortie convergent vers l'équipage mobile (33), ledit véhicule pouvant être rapproché de l'équipage mobile pour diminuer la quantité de lumière entrant dans ledit équipage par défocalisation de ladite lentille.

**Patentansprüche**

1. Chirurgische, insbesondere ophtalmologische Behandlungsvorrichtung, umfassend:
   – mindestens eine erste, ein Leistungsstrahlenbündel (23) längs einer ersten optischen Achse (1) und in einem vorbestimmten Querschnitt (34) emittierende Lichtquelle (230),
   – eine zweite, ein sichtbares Strahlenbündel (29) längs einer zweiten optischen Achse (2) emittierende Lichtquelle (28),
   – ein um die zweite optische Achse drehbewegliches Gestell (33), das Mittel zum Transformieren des von der zweiten Quelle emittierten Strahlenbündels in mindestens einen Elementarstrahl (30) umfaßt, der um die zweite optische Achse umläuft und angeordnet ist zum Bilden eines rohrförmigen Lichtstrahlenbündels (32), bestimmt zum Umhüllen des Leistungsstrahlenbündels,
   – Mittel (9, 27) zum Kombinieren des Leistungsstrahlenbündels und des rohrförmigen Lichtstrahlenbündels längs einer dritten gemeinsamen optischen Achse (3), und
   – eine im Strahlengang der dritten optischen Achse angeordnete Linse (41) zum Konzentrieren des Leistungsstrahlenbündels und des dieses umhüllenden rohrförmigen Lichtstrahlenbündels in einen Brennpunkt (12) der Linse gemäß einem Konuswinkel mit vorbestimmter Spitze,
   dadurch gekennzeichnet, daß die einen Teil des beweglichen Gestells bildenden Mittel mindestens einen homogenen Lichtleiter (21) umfassen,

2. Vorrichtung nach Anspruch 1, dadurch gekennzeichnet, daß der homogene Lichtleiter eine optische Faser ist.

3. Vorrichtung nach Anspruch 1, dadurch gekennzeichnet, daß das bewegliche Gestell Mittel zum Transformieren des von der zweiten Quelle emittierten Strahlenbündels in zwei Elementarstrahlen (30, 31) umfaßt und die genannten Mittel zwei homogene Lichtleiter umfassen.

4. Vorrichtung nach Anspruch 3, dadurch gekennzeichnet, daß die homogenen Lichtleiter gekuppelte optische Fasern sind.

5. Vorrichtung nach Anspruch 3, dadurch gekennzeichnet, daß die homogenen Lichtleiter Kanäle (25, 26, 36) sind, erhalten durch Dotieren einer Glasplatte (35).

6. Chirurgische, insbesondere ophtalmologische Behandlungsvorrichtung, umfassend:
   – mindestens eine erste, ein Leistungsstrahlenbündel (23) längs einer ersten optischen Achse (1) und in einem veränderbaren Querschnitt (34) emittierende Lichtquelle (230),
   – eine zweite, ein sichtbares Strahlenbündel (29) längs einer zweiten optischen Achse (2) emittierende Lichtquelle (28),
   – ein um die zweite optische Achse drehbewegliches Gestell (33), das Mittel zum Transformieren des von der zweiten Quelle emittierten Strahlenbündels in mindestens einen Elementarstrahl (30) umfaßt, der um die zweite optische Achse umläuft und angeordnet ist zum Bilden eines rohrförmigen Lichtstrahlenbündels (32), bestimmt zum Umhüllen des Leistungsstrahlenbündels,
   – Mittel (9, 27) zum Kombinieren des Leistungsstrahlenbündels und des rohrförmigen Lichtstrahlenbündels längs einer dritten gemeinsamen optischen Achse (3), und
   – eine im Strahlengang der dritten optischen Achse angeordnete Linse (41) zum Konzentrieren des Leistungsstrahlenbündels und des dieses umhüllenden rohrförmigen Lichtstrahlenbündels in einen Brennpunkt (12) der Linse gemäß einem Konuswinkel mit vorbestimmter Spitze,
   dadurch gekennzeichnet, daß die ersten, einen Teil des beweglichen Gestells bildenden Mittel mindestens einen homogenen Lichtleiter (21) umfassen und das bewegliche Gestell ferner zweite Mittel umfaßt, ausgebildet zum Verändern des Durchmessers ($D_2$), den das rohrförmige Lichtstrahlenbündel aufweist, derart, daß es das Leistungsstrahlenbündel entsprechend dem für das Leistungsstrahlenbündel gewählten Querschnitt ($D_1$) umhüllt.

7. Vorrichtung nach Anspruch 6, dadurch gekennzeichnet, daß das bewegliche Gestell erste Mittel zum Transformieren des von der zweiten Quelle emittierten Strahlenbündels in zwei Elementarstrahlen (30, 31) umfaßt, welche Mittel zwei gekuppelte, die Form eines Y aufweisende optische Fasern umfassen, und daß die zweiten Mittel eine Anordnung umfassen, die es ermöglicht, die beiden oberen Zweige (49, 50) des Y einander beidseits der zweiten optischen Achse zu nähern oder ihren Abstand zu vergrößern.

8. Vorrichtung nach Anspruch 7, dadurch gekennzeichnet, daß die Anordnung zwei, je ein Ende einer einen oberen Zweig des Y bildenden optischen Faser tragende Schlitten (52, 53) umfaßt, die in einer Gleitführung (40) verschieblich sind unter der Wirkung einer Endlosschraube (40) mit in der Mitte derselben wechselndem Steigungssinn, um die Schlitten voneinander zu entfernen oder einander zu nähern, wenn man die Endlosschraube in einer ersten bzw. zweiten Richtung dreht.

9. Vorrichtung nach Anspruch 6, dadurch gekennzeichnet, daß das bewegliche Gestell erste Mittel umfaßt zum Transformieren des von der zweiten Quelle emittierten Strahlenbündels in ein erstes (60, 61) und ein zweites (70, 71) Paar von Elementarstrahlen, welche ersten Mittel einen homogenen Lichtleiter umfassen, der sich baumartig verzweigt zum Bilden, wenn er von dem drehbeweglichen Gestell angetrieben wird, eines ersten und eines zweiten, koaxialen rohrförmigen Lichtstrahlenbündels mit unterschiedlichen Durchmessern, und daß die zweiten Mittel ein Element umfassen, das die Auswahl des einen oder des andern paares von Elementarstrahlen ermöglicht.

10. Vorrichtung nach Anspruch 9, dadurch gekennzeichnet, daß der sich baumartig verzweigende homogene Lichtleiter von optischen Fasern (64) gebildet ist.

11. Vorrichtung nach Anspruch 9, dadurch gekennzeichnet, daß der sich baumartig verzweigende homogene Lichtleiter von Kanälen (85) gebildet ist, erhalten durch Dotieren einer Glasplatte (86).

12. Vorrichtung nach Anspruch 9, dadurch gekennzeichnet, daß das Auswahlelement eine Lochplatte (79) umfaßt, die in einer ersten Position, in der sie das erste Elementarstrahlenpaar passieren läßt, oder in einer zweiten Position, in der sie das zweite Elementarstrahlenpaar passieren läßt, angeordnet werden kann.

13. Vorrichtung nach Anspruch 1 oder Anspruch 6, dadurch gekennzeichnet, daß zwischen der zweiten Lichtquelle und dem beweglichen Gestell ein Einsteller (90, 92) angeordnet ist, der die Intensität des von dem oder den Elementarstrahl(en) emittierten Lichts zu variieren ermöglicht.

14. Vorrichtung nach Anspruch 13, dadurch gekennzeichnet, daß der Einsteller ein Schlitten (92) ist, der eine Linse (90) trägt, deren Ausgangsstrahlen in Richtung auf das bewegliche Gestell (33) konvergieren, welcher Schlitten an das bewegliche Gestell annäherbar ist, um die in das Gestell eintretende Lichtmenge durch Defokalisierung der Linse zu verringern.

## Claims

1. Surgical treatment device, in particular for ophtalmological purposes comprising :
– at least one first light source (230) emitting a power beam (23) along a first optical axis (1) and having a predetermined cross-section (34),
– a second light source (28) emitting a visible light beam (29) along a second optical axis (2),
– a rotating element (33) rotating around said second optical axis, said element including means for transforming the beam issuing from said second source into at least one elementary beam (30) turning around said second optical axis and arranged to form a luminous tubular beam (32) intended to surround said power beam,
– means (9, 27) for combining said power beam and said luminous tubular beam along a third common optical axis (3), and
– a lens (41) arranged in the path of the third optical axis so as to concentrate said power beam and said luminous tubular beam which surrounds it onto the focal point (12) of the lens in accordance with a cone having a predetermined vertex angle,
characterized by the fact that said means forming part of said rotating element include at least one homogeneous light guide (21).

2. Device according to claim 1, characterized by the fact that the homogeneous light guide comprises an optical fibre.

3. Device according to claim 1, characterized by the fact that said rotating element includes means for transforming the beam issuing from said second source into two elementary beams (30, 31) and that said means include two homogeneous light guides.

4. Device according to claim 3, characterized by the fact that the homogeneous light guides comprise coupled optical fibres.

5. Device according to claim 3, characterized by the fact that the homogeneous light guides comprise channels (25, 26, 36) obtained by doping a glass plate (35).

6. Surgical treatment device, in particular for ophtalmological purposes, comprising :
– at least one first light source (230) emitting a power beam (23) along a first optical axis (1) and which has a cross-section (34) which may be varied,
– second light source (28) emitting a visible light beam (29) along a second optical axis (2),
– a rotating element (33) rotating around said second optical axis, said element including first means for transforming the beam issuing from said second source into at least one elementary beam (30) turning around said second optical axis and arranged to form a luminous tubular beam (32) intended to surround said power beam,
– means (9, 27) for combining said power beam and said luminous tubular beam along a third common optical axis (3), and
– a lens (41) arranged in the path of said third optical axis for concentrating said power beam and said luminous tubular beam which surrounds it onto the focal point (12) of the lens in accordance with a cone having a predetermined vertex angle,

characterized by the fact that the first means forming part of the rotating element include at least one homogeneous light guide (21), and that said rotating element further comprises second means adapted for varying the diameter ($D_2$) exhibited by the luminous tubular beam in a manner such that it surrounds the power beam in correspondance with the cross-section ($D_1$) chosen for said power beam.

7. Device according to claim 6, characterized by the fact that the rotating element includes first means for transforming the beam issuing from said second source into two elementary beams (30, 31), said first means comprising two coupled optical fibres (49, 50, 51) exhibiting the form of a Y and that the second means includes an arrangement enabling the bringing together or the spreading apart of the two upper branches (49, 50) of the Y from one another about said second optical axis.

8. Device according to claim 7, characterized by the fact that said arrangement includes two transporters (52, 53) each bearing the end of an optical fibre forming an upper branche of the Y, the transporters being adapted to slide in a guideway (40) under the urging of an endless screw (45) having its threading reversed midway along the length thereof in order to spread apart or bring together said transporters relative to one another according to whether the endless screw is rotated respectively in a first or in a second sense.

9. Device according to claim 6, characterized by the fact that the rotating element includes first means for transforming the beam issuing from the second source into first (60, 61) and second (70, 71) pairs of elementary beams, said first means comprising a homogeneous light guide developing in a treelike manner so as to form when driven in rotation by the rotating element, first and second coaxial luminous tubular beams of different diameters and said second means include an element enabling the selection of one or the other of said pairs of elementary beams.

10. Device according to claim 9, characterized by the fact that the homogeneous light guide developing in a treelike manner is formed by optical fibres (64).

11. Device according to claim 9, characterized by the fact that the homogeneous light guide developing in a treelike manner is formed by channels (85) obtained by doping a glass plate (86).

12. Device according to claim 9, characterized by the fact that said selection element includes a plate (79) having holes therein adapted to be arranged in a first position in which it allows passage of the first pair of elementary beams or in a second position in which it allows passage of the second pair of elementary beams.

13. Device according to claim 1 or claim 6, characterized by the fact that between the second light source and the rotating element there is placed an attenuator (90, 92) enabling varying of the light intensity of the elementary beam or beams.

14. Device according to claim 13, characterized by the fact that the attenuator is a transporter (92) bearing a lens (90) the output rays of which converge toward the rotating element (33), said transporter being adapted to approach the rotating element in order to diminish the quantity of light entering said element by defocussing said lens.

Art antérieur

FIG. 1

EP 0 362 764 B1

FIG. 2

FIG. 3

FIG. 5

FIG. 4

EP 0 362 764 B1

FIG. 7

FIG. 6

EP 0 362 764 B1

13

FIG. 9

FIG. 8

EP 0 362 764 B1

FIG. 10

FIG. 11

FIG. 12